(19) Europäisches Patentamt
European Patent Office
Office européen des brevets

(11) **EP 4 153 263 B1**

(12) **EUROPÄISCHE PATENTSCHRIFT**

(45) Veröffentlichungstag und Bekanntmachung des Hinweises auf die Patenterteilung:
**13.09.2023  Patentblatt 2023/37**

(21) Anmeldenummer: **22728557.4**

(22) Anmeldetag: **10.05.2022**

(51) Internationale Patentklassifikation (IPC):
*A61M 1/16* (2006.01)     *B01D 61/28* (2006.01)
*A61M 1/36* (2006.01)

(52) Gemeinsame Patentklassifikation (CPC):
**A61M 1/1654; A61M 1/1605; A61M 1/1607;**
**A61M 1/1609; A61M 1/1656; A61M 1/3609;**
**A61M 1/361; A61M 1/3612**

(86) Internationale Anmeldenummer:
**PCT/EP2022/062696**

(87) Internationale Veröffentlichungsnummer:
**WO 2022/238435 (17.11.2022 Gazette 2022/46)**

(54) **ELEKTROLYTBILANZIERUNGSMODUL**

ELECTROLYTE-BALANCING MODULE

MODULE D'ÉTABLISSEMENT DE BILAN ÉLECTROLYTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.05.2021  DE 102021112322**

(43) Veröffentlichungstag der Anmeldung:
**29.03.2023  Patentblatt 2023/13**

(73) Patentinhaber: **B. Braun Avitum AG**
**34212 Melsungen (DE)**

(72) Erfinder: **JANIK, Waldemar**
**34212 Melsungen (DE)**

(74) Vertreter: **Winter, Brandl - Partnerschaft mbB**
**Alois-Steinecker-Straße 22**
**85354 Freising (DE)**

(56) Entgegenhaltungen:
EP-A2- 0 097 366         WO-A1-2020/181220
US-A1- 2012 018 379     US-A1- 2019 275 230
US-B2- 10 918 775

• UWE KUHLMANN ET AL: "Zero Diffusive Sodium Balance in Hemodialysis Provided by an Algorithm-Based Electrolyte Balancing Controller: A Proof of Principle Clinical Study", ARTIFICIAL ORGANS, BLACKWELL SCIENTIFIC PUBLICATIONS, INC., BOSTON, US, Bd. 43, Nr. 2, 27. September 2018 (2018-09-27), Seiten 150-158, XP071485696, ISSN: 0160-564X, DOI: 10.1111/AOR.13328 in der Anmeldung erwähnt

**Beschreibung**

Die vorliegende Offenbarung betrifft eine Vorrichtung zur Elektrolytbilanzregelung einer Dialysierflüssigkeit bei einer extrakorporalen Blutbehandlung.

Bei der extrakorporalen Blutbehandlung (z.B. Hämodialyse) wird das Blut des Patienten von der sogenannten Dialysierflüssigkeit in einem Dialysator umspült. Die Dialysierflüssigkeit besteht u.a. aus hochreinem Wasser, einer ersten basischen Komponente (Natriumhydrogencarbonat ($NaHCO_3$)) sowie einer zweiten sauren Komponente. Letztere setzt sich üblicherweise aus Natriumchlorid (NaCl), Kaliumchlorid (KCl), Magnesiumchlorid ($MgCl_2$), Calciumchlorid ($CaCl_2$), Essigsäure ($CH_3COOH$) und Glucose zusammen. In der Regel werden zur Herstellung bzw. Proportionierung der Dialysierflüssigkeit Dosierpumpen und Leitfähigkeitssonden eingesetzt. Eine Sonde misst dabei die Leitfähigkeit nach Zugabe des Natriumhydrogencarbonats mittels einer ersten Dosierpumpe. Eine weitere Sonde erfasst die Leitfähigkeit der gesamten Dialysierflüssigkeit, nachdem auch die saure Komponente mittels einer weiteren Dosierpumpe hinzugegeben worden ist. Anhand der gemessenen Leitfähigkeiten werden dann die einzelnen Zugabemengen geregelt. Dieses Verfahren ist bekannt als die leitfähigkeitsgesteuerte Proportionierung und basiert im Wesentlichen auf dem Grundprinzip einer Rückkopplungsregelung (Feedback-Regelung).

Bei der volumetrischen Proportionierung dagegen dienen die Leitfähigkeitssonden lediglich zur Kontrolle. Die Proportionierung erfolgt hier direkt über die Dosierpumpenförderraten, was die Kenntnis bzgl. der Zusammensetzung der eingesetzten Komponenten erfordert. Dieses Verfahren, kommt somit ohne Rückkopplung auf Messergebnisse und entsprechender Nachjustierung der Pumpen aus und entspricht somit dem Grundprinzip einer Vorwärtsteuerung (Feedforward-Steuerung).

Die richtige Zusammensetzung der Dialysierflüssigkeit ist für das Wohlbefinden und die Lebenserwartung eines Patienten unabdingbar. Insbesondere Natrium spielt hierbei eine große Rolle, da es sowohl in der Dialysierflüssigkeit als auch im Blutplasma das am häufigsten vorkommende Kation ist. Bei niereninsuffizienten Patienten ist die Regulierung des Wasser- und des Elektrolythaushalts über die Nieren stark eingeschränkt. Aus diesem Grund sind die Patienten darauf angewiesen, ihre Wasser- und Natriumaufnahme auf ein Mindestmaß zu reduzieren. Natrium wird insbesondere in Form von Kochsalz (NaCl) aufgenommen. Eine erhöhte Natriumaufnahme gleicht ein Dialysepatient aus, indem er eine entsprechende Menge Flüssigkeit aufnimmt. Eine erhöhte Flüssigkeitsaufnahme führt allerdings wiederum dazu, dass dem Patienten während der nächsten Dialysebehandlung mehr überschüssige Flüssigkeit durch Ultrafiltration entzogen werden muss.

Studien haben allerdings gezeigt, dass eine erhöhte Ultrafiltrationsrate mit einer höheren Sterblichkeitsrate einhergeht (Flythe, J. E.; Kimmel, S. E., Brunelli, S. M.: "Rapid fluid removal during dialysis is associated with cardiovascular morbidity and mortality", Kidney International, 2010, 79, 250-257). Das bedeutet, dass diese Form der Patientenbehandlung möglichst behutsam durchgeführt werden sollte.

Während der Dialyse können hohe Ultrafiltrationsraten zudem symptomatische Blutdruckabfälle verursachen (Saran, R.; Bragg-Gresham, J.; Levin, N.; Twardowski, Z.; Wizemann, V.; Saito, A.; Kimata, N., Gillespie, B.: "Longer treatment time and slower ultrafiltration in hemodialysis: Associations with reduced mortality in the DOPPS", Kidney International, 2006, 69, 1222-1228).

Um letztere zu behandeln oder zu vermeiden, werden dem Patienten NaCl-Injektionen verabreicht oder der Patient wird gegen eine bzgl. Natrium recht hoch konzentrierte Dialysierflüssigkeit dialysiert. Allerdings führt beides wieder zu einer erhöhten Wasseraufnahme. Damit entsteht ein sich selbst verstärkender Kreislauf.

Ein zu geringer Natriumwert in der Dialysierflüssigkeit ist für den Patienten allerdings auch nicht besonders vorteilhaft. Hier sei darauf hingewiesen, dass ein Patientenkörper überschlägig in zwei "Compartments" (Räume) aufgeteilt werden kann, nämlich einen intrazellulären und einen extrazellulären Raum.

Der intrazelluläre und der extrazelluläre Raum stehen im Patienten in einem osmotischen Gleichgewicht zueinander. Eine geringe Natrium-Konzentration in der Dialysierflüssigkeit würde jedoch aufgrund von Diffusionsprozessen innerhalb des Dialysators zunächst zu einem (schnellen) Absinken der extrazellulären Osmolarität führen. Da aber beide Räume im Gleichgewicht stehen, würde Wasser aus dem extrazellulären Raum (nachträglich) in den intrazellulären Raum strömen. Die Zellen würden infolgedessen anschwellen, was zu Schwindel, Kopfschmerzen, Übelkeit, Krämpfen und ebenfalls zu Blutdruckabfällen führen kann.

Aus den vorstehend genannten Gründen ist es deshalb erstrebenswert, die Natrium-Konzentration in der Dialysierflüssigkeit individuell derart anzupassen, dass die Plasma-Natrium-Konzentration des Patienten während der Behandlung möglichst wenig und bestenfalls gar nicht geändert wird. Solch eine Dialyse wird als "isonaträmisch" bezeichnet (de Paula, F. M.; Peixoto, A. J.; Pinto, L. V.; Dorigo, D.; Patricio, P. J. M., Santos, S. F. F.: "Clinical consequences of an individualized dialysate sodium Prescription in hemodialysis patients", Kidney International, 2004, 66, 1232-1238; und Basile, C., Lomonte, C.: "It is Time to Individualize the Dialysate Sodium Prescription", Seminars in Dialysis, 2016, 29, 24-27).

Am einfachsten wäre es, dem Patienten vor jeder Behandlung eine Blutprobe zu entnehmen und zu analysieren, um die Natrium-Konzentration in der Dialysierflüssigkeit auf dieser Basis einzustellen. Allerdings ist diese Vorgehensweise sehr zeitintensiv und kostspielig sowie mit einem hohen apparativen Aufwand verbunden. Aus diesem Grund wird in vielen Dialysezentren mit einer standardisierten Zusammensetzung der Dialysierflüssigkeit dialysiert, obwohl die Plasma-Natrium Konzentration von Patient zu Patient unterschiedlich ausfallen kann (Mendoza, J. M.; Sun, S.; Chertow, G. M.; Moran, J.; Dass, S., Schiller, B.: "Dialysate Sodium and Sodium gradient in maintenance hemodialysis: a neglected sodium restriction approach?",Nephrol Dial Transplant, 2011, 26, 1281-1287).

Stand der Technik

Es sind Lösungen bekannt, die Natrium-Konzentration bei der Dialyse zu messen.

Eine Dialysevorrichtung mit geregelter Zusammensetzung der Dialysierflüssigkeit wird in der EP 0 097 366 A2 offenbart. Bei dieser Dialysevorrichtung wird der Elektrolytgehalt der Dialysierflüssigkeit bevor diese in den Dialysator eintritt und nachdem sie den Dialysator wieder verlässt mittels zweier Detektoren erfasst und wird basierend auf dem Unterschied der erhaltenen Messwerte die Zusammensetzung der Dialysierflüssigkeit geregelt. Sinn und Zweck dieser Maßnahme ist es, ein Elektrolyt-Konzentrationsgleichgewicht zwischen Dialysierflüssigkeit und Patientenblut herzustellen, um einen Elektrolytentzug aus dem Patientenblut zu vermeiden

Eine Dialysevorrichtung zur Durchführung einer isonaträmischen Dialyse und ein Verfahren zur Natriumbilanzierung im Dialysierflüssigkeitskreislauf ist aus der WO 2010 / 112 223 A1 bekannt. Im Speziellen wird in einem Dialysierflüssigkeitskreislauf die Leitfähigkeit der Dialysierflüssigkeit vor und nach dem Dialysator bestimmt und miteinander verglichen. Als weitere Parameter werden zudem die Konzentrationen von Bicarbonat und Kalium im Blut des Patienten benötigt, die zuvor mithilfe eines externen Messgerätes bestimmt werden müssen.

Kuhlmann, U.; Maierhofer, A.; Canaud, B.; Hoyer, J., Gross, M., "Zero Diffusive Sodium Balance in Hemodialysis Provided by an Algorithm-Based Electrolyte Balancing Controller: A Proof of Principle Clinical Study", Artificial Organs, Wiley, 2018, beschreiben einen Algorithmus zur Steuerung der Natriumkonzentration in der Dialysierflüssigkeit einer Hämodialyse oder Hämodiafiltration basierend auf Leitfähigkeitsmessungen der Dialysierflüssigkeit vor und nach dem Dialysator.

DE 10 2017 116 097 A1 offenbart eine Dialysevorrichtung bei der eine Plasma-Natrium-Konzentration des Blutes gemessen wird. Dabei wird die Zusammensetzung der Dialysierflüssigkeit in Abhängigkeit von der gemessenen Konzentration derart eingestellt, dass die Plasma-Natrium-Konzentration am Ende der Blutbehandlung den gleichen Wert aufweist, wie zu Beginn.

Nachteile des Stands der Technik

Der Stand der Technik sieht Lösungen vor, die auf bereits vorhandene Bestandteile der Dialysemaschine zurückgreifen. Dazu zählt neben den maschinenseitigen Leitfähigkeitssonden vor allem die Software, in welcher der Natrium-Steuerungs- bzw. Regelungsalgorithmus implementiert sein muss. Änderungen bzw. Erweiterungen der Maschinensoftware sind allerdings mit einem hohen Aufwand verbunden und darüber hinaus sehr kostenintensiv. Des Weiteren verfügen nicht alle Maschinen über die notwendige Sensorik am Ein- und/oder Ausgang des Dialysators.

Zusammenfassung der Offenbarung

Der Erfindung liegt angesichts dieser Problematik die Aufgabe zugrunde, eine Maßnahme bereit zu stellen, mittels welcher eine Dialysemaschine auf einfache Weise derart veränderbar ist, dass eine Elektrolytbilanzierung möglichst unabhängig von der bereits vorhandenen Hard- und/oder Software der jeweiligen Dialysemaschine ermöglicht wird.

Offenbarungsgemäß wird diese Aufgabe durch eine externe/separate/autarke Vorrichtung zur Elektrolytbilanzregelung einer Dialysierflüssigkeit bei einer extrakorporalen Blutbehandlung nach Anspruch 1 gelöst, die folglich als separat/unabhängig arbeitende Einheit an eine Dialysemaschine (adaptiv) angebracht werden kann. In anderen Worten ausgedrückt betrifft die vorliegende Erfindung eine separate, im Wesentlichen autark von Soft- und/oder Hardware der Dialysemaschine arbeitende/funktionierende Vorrichtung zur Elektrolytbilanzierung, die nachträglich an eine bereits vorhandene Maschine (adaptiv) anbringbar ist und somit eine bekannte Dialysemaschine erweitert/nachrüstet, ohne dass hierfür Software- und/oder Hardware-Änderungen an der Dialysemaschine vorgenommen werden müssen. Dies bedeutet letztlich, dass vorzugsweise alle für eine solche Elektrolytbilanzierung notwendigen Bauteile (einschließlich Sensorik, Steuerung vorzugsweise mit eigener, autark funktionierender Software, ggf. Anzeige und optional sogar Energieversorgung bzw. Energieanschlussmöglichkeit) in der erfindungsgemäßen Vorrichtung vorhanden sind, sodass ein Rückgriff auf Einrichtungen/Software der Dialysemaschine ggf. möglich aber nicht notwendig ist.

Vorteilhafte Weiterbildungen der Offenbarung sind Gegenstand der beigefügten Unteransprüche.

Die vorliegende Offenbarung betrifft konkreter ausgedrückt eine Vorrichtung zur Elektrolytbilanzierung bei einer extrakorporalen Blutbehandlung, insbesondere einer Hämodialyse. Die Vorrichtung weist eine Sensoreinheit auf, mit

- mindestens einer ersten Messvorrichtung zum Erfassen eines Natriumgehalts einer verbrauchten Dialysierflüssigkeit,
- einem Anschlussabschnitt oder -teil zum Anschließen der Sensoreinheit an einen Fluidkreis einer Blutbehandlungsmaschine, derart, dass die Messvorrichtung zum Erfassen des Natriumgehalts in Funktion gesetzt ist und
- eine Elektronik- und Auswerteeinheit, die den von der ersten Messvorrichtung erfassten Natriumgehalt analysiert und in Abhängigkeit des erfassten Natriumgehalts eine Empfehlung für einen Natriumgehalt einer frischen Dialysierflüssigkeit ermittelt. Ferner hat die Vorrichtung
- eine (eigene/interne), vorzugsweise kombinierte Eingabe-/Ausgabeschnittstelle (nachfolgend einfach als Bedieneinheit bezeichnet), die einem Benutzer die von der Elektronik- und Auswerteeinheit ermittelte Empfehlung für den Natriumgehalt der frischen Dialysierflüssigkeit ausgibt bzw. anzeigt.

Die Übernahme der ausgegebenen Empfehlung in den programmierten Ablauf der Blutbehandlungsmaschine erfolgt dann händisch durch den Bediener.

Offenbarungsgemäß erweitert die erfindungsgemäß als Insellösung konzipierte Vorrichtung eine extrakorporale Blutbehandlungsmaschine, wie Dialysemaschine, auf der die extrakorporale Blutbehandlung abläuft und führt unabhängig zur Blutbehandlung ein Programm mit den folgenden Schritten aus:

- Initialisieren einer Bestimmung des Natriumgehalts der verbrauchten Dialysierflüssigkeit bzw. einer mit der Plasma-Natrium-Konzentration im Blut des Patienten korrelierten Größe durch den Benutzer/Bediener über die Bedieneinheit;
- Erfassen des Natriumgehalts der verbrauchten Dialysierflüssigkeit durch die Messvorrichtung;
- Anweisen des Benutzers/Bedieners, die Dialysemaschine in einen Bypassmodus zu schalten;
- Informieren der Vorrichtung über den Bypassmodus beispielsweise durch selbständiges sensorisches Erkennen oder durch händische Eingabe an der Bedieneinheit;
- Anweisen des Benutzers, die Dialysemaschine in einen Normalbetriebsmodus zu schalten;
- Informieren der Vorrichtung über den Normalbetriebsmodus beispielsweise durch selbständiges sensorisches Erkennen oder durch händische Eingabe an der Bedieneinheit;
- Erfassen des Natriumgehalts der verbrauchten Dialysierflüssigkeit durch die Messvorrichtung;
- Analyse des erfassten Natriumgehalts in der verbrauchten Dialysierflüssigkeit und Ausgabe der Empfehlung eines Natriumgehalts der frischen Dialysierflüssigkeit in Abhängigkeit des erfassten Natriumgehalts in der verbrauchten Dialysierflüssigkeit;
- Anweisung an den Benutzer, den empfohlenen Natriumgehalt an der Dialysemaschine händisch einzustellen;

In dem Bypassmodus fließt während der Blutbehandlung kurzzeitig keine Dialysierflüssigkeit durch einen Dialysator. Die Dialysierflüssigkeit wird durch ein Bypassventil abgeleitet, umgeht den Dialysator und wird zu einem Abfluss geleitet. Das bewirkt, dass im Dialysator während des Bypasses nur wenig Dialysierflüssigkeit vorliegt. Die Dialysierflüssigkeit nimmt dadurch Natrium aus dem Blut bis zu einer Sättigung derart auf, dass die Natriumkonzentration in der Dialysierflüssigkeit weitestgehend der Natriumkonzentration im Blut entspricht. Dadurch kann im Bypassmodus die Natriumkonzentration im Blut gemessen werden, ohne auf der Blutkreislaufseite des Dialysevorgangs messen zu müssen. Dieses Verfahren ist aus der DE 10 2017 116 097 A1 bekannt.

Der Normalbetriebsmodus ist der Modus, der nicht der Bypassmodus ist, und in dem die frische Dialysierflüssigkeit von der Dialysemaschine durch die Messvorrichtung in den Dialysator und verbrauchte Dialysierflüssigkeit von dem Dialysator durch die Messvorrichtung fließt.

In anderen Worten ausgedrückt wird eine Vorrichtung bereitgestellt, die eine Dialysemaschine erweitert. Dabei weist die Vorrichtung eine Sensorik auf, die den Natriumgehalt in zumindest einer Leitung der Dialysemaschine misst. Die Vorrichtung hat weiterhin eine Auswerteeinheit, die die Messwerte der Sensorik verarbeitet. Dabei sind die Sensorik und die Auswerteeinheit derart ausgebildet, dass die Vorrichtung selbständig eine Elektrolytbilanzierung durchführt. Die Auswerteeinheit ermittelt einen Soll-Natriumwert in der Dialysierflüssigkeit basierend auf den Messwerten der Sensorik. Ferner weist die Vorrichtung zur Elektrolytbilanzierung auch eine Bedieneinheit auf. Die Bedieneinheit hat eine graphische Benutzeroberfläche. Auf der Bedieneinheit mit der graphischen Benutzeroberfläche wird dem Benutzer das Ergebnis der Analyse der Elektronik- und Auswerteeinheit dargestellt. Für diese Funktionen muss die Vorrichtung nicht auf die Hardware und/ oder die Software der Dialysemaschine zugreifen. Dadurch kann die Vorrichtung die Dialysemaschine erweitern, ohne dass Änderungen der Dialysemaschinensoftware notwendig sind.

Die Vorrichtung führt das folgende Programm aus:

Durch eine Betätigung der Bedieneinheit des Elektrobilanzierungsmoduls wird eine Bestimmung einer chemischen oder physikalischen Größe, die mit dem Natriumgehalt einer verbrauchten Dialysierflüssigkeit korreliert, initialisiert. Die Messvorrichtung erfasst den Natriumgehalt der verbrauchten Dialysierflüssigkeit für eine bestimmte Zeitdauer. Die Bedieneinheit der Vorrichtung gibt eine Anweisung an den Benutzer aus, die Dialysemaschine in einen Bypassmodus zu schalten. Der Benutzer schaltet die Dialysemaschine an einer Bedienoberfläche der Dialysemaschine in den Bypassmodus. Die Vorrichtung erkennt, dass sich die Dialysemaschine in dem Bypassmodus befindet. Die Vorrichtung kann den Bypassmodus entweder durch einen Flusssensor in der Sensoreinheit erkennen oder dadurch, dass der Benutzer manuell einträgt, dass sich die Dialysemaschine in dem Bypassmodus befindet. Nach einer definierten Zeitdauer gibt die Vorrichtung über die Bedieneinheit ein Signal aus, die Dialysemaschine wieder zurück in den Hauptschluss/ Normalbetriebsmodus zu schalten. Die Dialysemaschine wird daraufhin von dem Benutzer manuell durch Betätigung der Bedienoberfläche der Dialysemaschine von dem Bypassmodus in den Hauptschluss/ Normalbetriebsmodus geschaltet. Durch den Flusssensor oder manuelle Bestätigung erkennt die Vorrichtung, dass der Bypassmodus beendet wurde. Daraufhin wird der Natriumgehalt der verbrauchten Dialysierflüssigkeit von der Messvorrichtung erfasst. Damit erfasst die Messvorrichtung eine chemische oder physikalische Größe, die mit dem Natriumgehalt einer verbrauchten Dialysierflüssigkeit korreliert. Die Elektronik- und Auswerteeinheit analysiert den erfassten Natriumgehalt und empfiehlt in Abhängigkeit des erfassten Natriumgehalts ein Natriumgehalt für die frische Dialysierflüssigkeit. Zuletzt wird der empfohlene Natriumgehalt über die Bedieneinheit an den Benutzer ausgegeben.

Die Vorrichtung ist separat/unabhängig von einer Dialysemaschine ausgebildet. Dadurch können bestehende Dialysemaschinen mit der Vorrichtung erweitert werden, ohne dass kostenintensive und aufwändige Änderungen in der Maschinensoftware vorgenommen werden müssen, da die Vorrichtung auf keine Maschinen-interne Bauteile zurückgreifen muss. Des Weiteren sind nicht alle vorhandenen Dialysemaschinen mit der notwendigen Sensorik für die Dialysierflüssigkeitsüberwachung ausgestattet. Diese Sensorik stellt die separate Vorrichtung zur Elektrolytbilanzierung bereit.

Bei einer vorteilhaften Ausgestaltung der Offenbarung, die in Kombination mit anderen Merkmalen oder auch alleinstehend ausgestalten sein kann, ist vorgesehen, dass die Sensoreinheit eine Messvorrichtung aufweist, die in einer Zuführleitung vorgesehen ist und einen Natriumgehalt der frischen Dialysierflüssigkeit erfasst.

In anderen Worten ausgedrückt, ist/wird in der Zuführleitung, die die frische Dialysierflüssigkeit zu dem Dialysator leitet, eine zweite Messvorrichtung/ Sensorik angebracht. Die zweite Messvorrichtung/ Sensorik erfasst den Natriumgehalt der frischen Dialysierflüssigkeit bzw. erfasst die temperaturkompensierte Leitfähigkeit der frischen Dialysierflüssigkeit. Die zweite Messvorrichtung/ Sensorik ist mit der Elektronik- und Auswerteeinheit verbunden und übermittelt die von der Messvorrichtung erfassten Messwerte an die Elektronik- und Auswerteeinheit.

Bei einer vorteilhaften Ausgestaltung der Offenbarung, die in Kombination mit anderen Merkmalen oder auch alleinstehend ausgestalten sein kann, ist vorgesehen, dass die erste Messvorrichtung und die zweite Messvorrichtung sowohl invasiv als auch nicht-invasiv messen können.

In anderen Worten ausgedrückt können die Messvorrichtungen entweder in Kontakt mit der Dialysierflüssigkeit stehen oder kontaktlos beispielsweise durch einen Gradiometer oder optisch messen.

Bei einer vorteilhaften Ausgestaltung der Offenbarung, die in Kombination mit anderen Merkmalen oder auch alleinstehend ausgestalten sein kann, ist vorgesehen, dass die Messvorrichtungen eine temperaturkompensierte Leitfähigkeit der Dialysierflüssigkeit bestimmen. Anders ausgedrückt, erfassen die Messvorrichtungen die temperaturkompensierte Leitfähigkeit und bestimmen anhand der temperaturkompensierten Leitfähigkeit den Natriumgehalt der Dialysierflüssigkeit in der jeweiligen Leitung.

Bei einer vorteilhaften Ausgestaltung der Offenbarung, die in Kombination mit anderen Merkmalen oder auch alleinstehend ausgestalten sein kann, ist vorgesehen, dass die die erste Messvorrichtung und die zweite Messvorrichtung eine Ionenkonzentration in der Dialysierflüssigkeit bestimmen.

Die Ionenkonzentration in der Dialysierflüssigkeit kann beispielsweise durch ionenselektive Elektroden oder optische Vorrichtungen zur Bestimmung von Ionenkonzentrationen wie die Laserinduzierte Plasmaspektroskopie bestimmt werden.

Bei einer vorteilhaften Ausgestaltung der Offenbarung, die in Kombination mit anderen Merkmalen oder auch alleinstehend ausgestalten sein kann, ist vorgesehen, dass die Vorrichtung eine dritte Messvorrichtung aufweist, die eine Flussrate der Dialysierflüssigkeit in der Zuführleitung bestimmt.

Anders ausgedrückt weist die Zuführleitung eine Messvorrichtung auf, die eine Fließgeschwindigkeit der Dialysierflüssigkeit in der Zuführleitung bestimmt. Alternativ kann die Messvorrichtung derart ausgebildet sein, dass sie lediglich feststellt, ob sich die Dialysierflüssigkeit bewegt. Die Messvorrichtung ist optional. Die Fließgeschwindigkeit der Dialysierflüssigkeit in der Zuführleitung kann durch einen Flussdetektor auf Ultraschallbasis erfasst werden.

Durch die Messung der Flussrate der Dialysierflüssigkeit in der Zuführleitung wird festgestellt, ob ein Bypassmodus der Dialysemaschine aktiv ist. Der Bypassmodus kann ebenfalls durch einen Hallsensor erkannt werden, der eine Stellung eines elektrischen Bypassventils in der Dialysemaschine erfasst. Der Bypassmodus ist ein Modus, indem eine Fluidverbindung zwischen der Zuführleitung und der Abführleitung besteht, um den Dialysator zu umgehen.

Bei einer vorteilhaften Ausgestaltung der Offenbarung, die in Kombination mit anderen Merkmalen oder auch alleinstehend ausgestalten sein kann, ist vorgesehen, dass die Empfehlung für den Natriumgehalt der Dialysierflüssigkeit derart gewählt wird, dass eine Plasma-Natrium-Konzentration im Blut eines Patienten über eine Dauer der Blutbehandlung im Wesentlichen gleichbleibt.

Anders ausgedrückt, soll die Plasma-Natrium-Konzentration im Blut des Patienten über die Dauer der Dialysebehandlung im Wesentlichen konstant bleiben. Deshalb wird eine Empfehlung für den Natriumgehalt der frischen Dialysierflüssigkeit derart gewählt, dass die Plasma-Natrium-Konzentration im Blut des Patienten konstant bleibt. Die Empfehlung für den Natriumgehalt der Dialysierflüssigkeit wird von der Elektronik- und Auswerteeinheit ermittelt und über die Bedieneinheit an den Nutzer ausgegeben.

Bei einer vorteilhaften Ausgestaltung der Offenbarung, die in Kombination mit anderen Merkmalen oder auch alleinstehend ausgestalten sein kann, ist vorgesehen, dass die Vorrichtung eine vierte Messvorrichtung aufweist, die eine chemische oder physikalische Größe, vorzugsweise eine Absorptionseigenschaft wie eine Extinktion, der verbrauchten Dialysierflüssigkeit in der Abführleitung bestimmt.

Für die vierte Messvorrichtung ist es auch denkbar einen enzymatischen oder elektrochemischen Sensor einzusetzen. Durch die Messung der Absorptionseigenschaft der verbrauchten Dialysierflüssigkeit wird ein Anteil an Toxinen und harnpflichtigen Stoffen in der verbrauchten Dialysierflüssigkeit bestimmt. Der Anteil an Toxinen und harnpflichtigen Stoffen wird beispielsweise dazu verwendet, den Einfluss von Stoffen, die nicht Natrium sind, auf die temperaturkompensierte Leitfähigkeit zu erfassen.

Bei einer vorteilhaften Ausgestaltung der Offenbarung, die in Kombination mit anderen Merkmalen oder auch alleinstehend ausgestalten sein kann, ist vorgesehen, dass die Messvorrichtungen einzeln direkt an den jeweiligen Leitungen angebracht werden.

In anderen Worten ausgedrückt, müssen die Messvorrichtungen nicht in einer einzigen Sensoreinheit zusammengefasst sein. Die einzelnen Messvorrichtungen können auch direkt an den jeweiligen, zu messenden Leitungen angebracht werden. Dabei kann die Leitung aufgetrennt und die Messvorrichtung eingebracht werden. Handelt es sich bei der Messvorrichtung um eine nicht-invasive arbeitende Messvorrichtung, muss die Leitung nicht aufgetrennt werden. Jede Messvorrichtung kann stattdessen von außen auf die Leitung angebracht werden. Dabei können alle Messvorrichtungen jeweils direkt an die jeweiligen Leitungen angebracht werden, oder es können auch nur einzelne der Messvorrichtungen direkt an die jeweilige Leitung angebracht werden.

Für die Elektrolytbilanzierung sind insbesondere die chemischen und/ oder physikalischen Größen in der verbrauchten Dialysierflüssigkeit der Abführleitung von Interesse. Deshalb reicht es in einem einfachsten Fall aus, lediglich die erste Messvorrichtung in der Abführleitung vorzusehen.

Bei einer vorteilhaften Ausgestaltung der Offenbarung, die in Kombination mit anderen Merkmalen oder auch alleinstehend ausgestalten sein kann, ist vorgesehen, dass die Sensoreinheit, die Elektronik- und Auswerteeinheit und die Bedieneinheit in einer Einheit zusammengefasst sind.

Die Sensoreinheit, die Elektronik- und Auswerteeinheit und die Bedieneinheit müssen nicht zwingend in einzelne Einheiten unterteilt sein. Vielmehr können die Sensoreinheit, die Elektronik- und Auswerteeinheit und die Bedieneinheit auch in eine gemeinsame Einheit/ in ein gemeinsames Gehäuse zusammengefasst sein. Ferner können auch einzelne der Sensoreinheit, der Elektronik- und Auswerteeinheit und der Bedieneinheit in Teileinheiten zusammengefasst werden. So kann beispielswiese die Sensoreinheit und die Elektronik- und Auswerteeinheit in eine gemeinsame Sensor-, Elektronik- und Auswerteeinheit zusammengefasst sein, die mit der Bedieneinheit verbunden ist.

Bei einer vorteilhaften Ausgestaltung der Offenbarung, die in Kombination mit anderen Merkmalen oder auch alleinstehend ausgestalten sein kann, ist vorgesehen, dass eine Verbindung zwischen der Elektronik- und Auswerteeinheit und der Bedieneinheit kabellos ausgebildet ist.

In anderen Worten ausgedrückt, ist die Verbindung zwischen der Elektronik- und Auswerteeinheit und der Bedieneinheit nicht auf ein Kabel reduziert. Die Verbindung kann auch durch eine kabellose Verbindung, beispielsweise WLAN oder Bluetooth realisiert sein.

Bei einer vorteilhaften Ausgestaltung der Offenbarung, die in Kombination mit anderen Merkmalen oder auch alleinstehend ausgestalten sein kann, ist vorgesehen, dass die Vorrichtung bei der Messung der temperaturkompensierten Leitfähigkeit eine Auswirkung von anderen leitenden Stoffen, die nicht Natrium sind, auf die gemessene Leitfähigkeit erfasst und die gemessene Leitfähigkeit entsprechend korrigiert.

Andere leitende Stoffe können die Leitfähigkeit der Flüssigkeit beeinflussen. Deshalb gibt es keinen direkten Zusammenhang zwischen der temperaturkompensierten Leitfähigkeit und dem Natriumgehalt. Die Vorrichtung bereinigt die gemessene temperaturkompensierte Leitfähigkeit um die Effekte von anderen leitenden Stoffen und Stoffen, die die Leitfähigkeit anderweitig beeinflussen. Toxine und andere harnpflichtige Stoffe mindern beispielsweise die Leitfähigkeit eines Fluides ohne selbst leitend zu sein.

Offenbarungsgemäß wird die Aufgabe der Offenbarung ferner durch ein Verfahren zur Elektrolytbilanzierung bei der extrakorporalen Blutbehandlung, insbesondere einer Hämodialyse, das auf der Vorrichtung gemäß einem der vorstehenden Aspekte anzuwenden ist, gelöst. Das Verfahren weist die folgenden Schritte auf:

Der erste Schritt ist das Initialisieren der Messung einer mit der Plasma-Natrium-Konzentration des Patientenbluts korrelierenden Größe durch Betätigung der Bedieneinheit, vorzugsweise innerhalb der ersten 15 Minuten. Danach folgt das Erfassen des Natriumgehalts an der Abführleitung und gegebenenfalls der Zuführleitung für eine definierte Zeitspanne. Danach erfolgt eine Aufforderung an der Bedieneinheit der Vorrichtung an den Bediener/Benutzer, die Dialysemaschine in den Bypassmodus zu schalten und der Bediener/Benutzer schaltet daraufhin die Dialysemaschine manuell durch die Bedienoberfläche der Dialysemaschine in den Bypass. Der nächste Schritt ist eine Aufforderung an der Bedieneinheit der Vorrichtung an den Bediener, die Dialysemaschine zurück in den Normalbetriebsmodus zu schalten. Darauf folgt eine manuelle Deaktivierung des Bypassmodus an der Bedienoberfläche der Dialysemaschine durch den Bediener. Erste Messvorrichtung und gegebenenfalls die zweite Messvorrichtung erfassen Natriumgehalt der verbrauchten Dialysierflüssigkeit für eine definierte Zeitspanne. Anschließend folgt eine Analyse des erfassten Natriumgehalts durch die Elektronik- und Auswerteeinheit und eine Empfehlung für den Natriumgehalt in der frischen Dialysierflüssigkeit in Abhängigkeit des erfassten Natriumgehalts. Die Empfehlung der Elektronik- und Auswerteeinheit wird durch die Bedieneinheit der Vorrichtung an den Bediener ausgegeben. Zuletzt erfolgt ein manuelles Eingeben des empfohlenen Soll- Natriumgehalts auf der Bedienoberfläche der Dialysemaschine durch den Bediener/ Benutzer.

Anders ausgedrückt, weist das Verfahren, das auf der Vorrichtung zur Elektrolytbilanzierung bei einer Dialyse ausgeführt wird, die folgenden Schritte auf. Die Bestimmung der Plasma-Natrium-Konzentration im Blut des Patienten wird initialisiert. Für eine definierte Zeit erfassen die Messvorrichtungen am Ausgang des Dialysators und gegebenenfalls am Eingang des Dialysators die jeweiligen Messwerte. Auf der Bedieneinheit erfolgt eine Aufforderung, die Dialysemaschine in den Bypassmodus zu schalten. Die Dialysemaschine wird über die Bedienoberfläche der Dialysemaschine manuell in den Bypassmodus geschaltet. Nach einer definierten Zeitspanne erscheint auf der Bedieneinheit die Aufforderung, die Dialyse wieder in den Hauptschluss/ Normalbetriebsmodus zurückzuschalten. Daraufhin wird der Bypassmodus über die Bedienoberfläche der Dialysemaschine manuell deaktiviert. Die Messvorrichtungen erfassen Messwerte für eine bestimmte Zeitdauer. Die Elektronik- und Auswerteeinheit analysiert die erfassten Messwerte und gibt eine Empfehlung für den Natriumgehalt in der frischen Dialysierflüssigkeit über die Bedieneinheit aus. Die Empfehlung für den Natriumgehalt in der frischen Dialysierflüssigkeit wird an der Bedienoberfläche der Dialysemaschine manuell eingegeben.

Offenbarungsgemäß wird die Aufgabe der Offenbarung durch ein Verfahren zur Anbringung einer Vorrichtung gemäß den vorstehenden Aspekten an der Dialysemaschine gelöst. Das offenbarungsgemäße Verfahren weist das Anbringen der Messvorrichtung an der Abführleitung der Dialysemaschine und gegebenenfalls das Anbringen der optionalen Messvorrichtung an der Abführleitung der Dialysemaschine auf. Weiter weist das Verfahren die Schritte gegebenenfalls Anbringen der Messvorrichtung an der Zuführleitung der Dialysemaschine und gegebenenfalls Anbringen der optionalen Messvorrichtung an der Zuführleitung der Dialysemaschine auf.

Bei einer vorteilhaften Ausgestaltung der Offenbarung, die in Kombination mit anderen Merkmalen oder auch alleinstehend ausgestalten sein kann, ist vorgesehen, dass die Messvorrichtung eine optische Messvorrichtung ist und außen an einer durchsichtigen Leitung angebracht wird.

Anders ausgedrückt, muss der Schlauch der Leitung für eine optische Messvorrichtung nicht getrennt werden. Die optische Messvorrichtung kann außen auf den Schlauch aufgesetzt werden, ohne den Schlauch/ die Leitung zu beschädigen.

Bei einer vorteilhaften Ausgestaltung der Offenbarung, die in Kombination mit anderen Merkmalen oder auch alleinstehend ausgestalten sein kann, ist vorgesehen, dass die Messvorrichtung invasiv an der Leitung angebracht wird, indem die Leitung in zwei Teile geschnitten wird, die Messvorrichtung mit dem ersten Teil verbunden wird, und die Messvorrichtung mit dem zweiten Teil verbunden wird. Damit steht die Messvorrichtung in direktem Kontakt mit der Flüssigkeit in der Leitung.

Bei einer vorteilhaften Ausgestaltung der Offenbarung, die in Kombination mit anderen Merkmalen oder auch alleinstehend ausgestalten sein kann, ist vorgesehen, dass die Vorrichtung eine Schnittstelle mit der Dialysemaschine hat. Durch diese Schnittstelle können Daten/ Informationen ausgelesen werden, die in der Dialysemaschine hinterlegt sind. Beispielsweise handelt es sich dabei um Daten, die die Blutbehandlung betreffen und/ oder patientenspezifische Daten. Beispielsweise handelt es sich dabei um das Gewicht oder das Geschlecht des Patienten. Besonders die patientenspezifischen Daten können verwendet werden um weitere Parameter wie einen spezifischen Kt/V-Wert zu berechnen. Es ist anzumerken, dass die Schnittstelle keine Änderungen in der Soft- und/oder Hardware der Dialysemaschine benötigt.

Bei einer vorteilhaften Ausgestaltung der Offenbarung, die in Kombination mit anderen Merkmalen oder auch alleinstehend ausgestalten sein kann, ist vorgesehen, dass die Vorrichtung durch die Sensoreinheit auch weitere Parameter erfasst, die vorstehend noch nicht genannt wurden. Diese Parameter sind beispielsweise der Kt/V-Wert, der eine Dialyseeffektivität angibt, oder der spezifische Kt/V-Wert. Es ist anzumerken, dass für eine Messung des Kt/V-Werts der optische Sensor, der den Gehalt an harnpflichtigen Stoffen erfasst, vorhanden sein muss und auch eine Möglichkeit bestehen muss, den Volumenstrom der Dialysierflüssigkeit zu ermitteln. Um den spezifischen Kt/V-Wert zu berechnen, müssen auch patientenspezifische Daten wie das Gewicht des Patienten vorhanden sein. Ferner könnte die Elektronik- und Auswerteeinheit basierend auf den Messwerten ermitteln, wie lange die Blutbehandlung noch dauert.

Vorzugsweise können die Ventile der Dialysemaschine, die für die bekannte Bypass-Schaltung benötigt werden, zu der Vorrichtung zur Elektrolytbilanzierung gehören. Dabei handelt es sich um drei Ventile. Ein erstes Ventil ist ein Eingangsventil zu dem Dialysator, ein zweites Ventil ist ein Ausgangsventil aus dem Dialysator. Ein drittes Ventil ist das Bypassventil, das die Dialysierflüssigkeit um den Dialysator herumfließen lässt. Im Bypassmodus sind das erste und das zweite Ventil geschlossen und das dritte Ventil ist offen. Das zweite Ventil muss dabei nicht zwangsläufig geschlossen sein. Dadurch umgeht die Dialysierflüssigkeit im Bypassmodus den Dialysator. Die verbleibende Dialysierflüssigkeit im Dialysator wird derart gesättigt, dass die die Natriumkonzentration in der Dialysierflüssigkeit weitestgehend der Natriumkonzentration im Blut entspricht. Dadurch kann im Bypassmodus die Natriumkonzentration im Blut gemessen werden, ohne auf der Blutkreislaufseite des Dialysevorgangs messen zu müssen.

Da die benötigten Ventile in der offenbarungsgemäßen Vorrichtung zur Elektrolytbilanzierung vorhanden sind, kann der Bypassmodus automatisch eingestellt werden, ohne dass der Benutzer den Bypassmodus händisch auf der Dialysemaschine starten oder beenden muss. Durch die Ventile in der (externen) Vorrichtung zur Elektrolytbilanzierung können auch (ältere) Dialysemaschinen, die weder die nötige Hardwareausstattung noch die nötige Software ohne aufwände Änderungen an der Hard- oder Software aufweisen, nachgerüstet werden.

Ferner kann die Vorrichtung zur Elektrolytbilanzierung zur Bestimmung einer Rezirkulationsrate genutzt werden. Dabei wird der Bypassmodus zweimal nacheinander eingestellt. Ein erstes Mal wird der Bypassmodus derart lange eingestellt, dass sich zwischen dem Blut und der (verbleibenden) Dialysierflüssigkeit ein Konzentrationsgleichgewicht einstellt. Ein zweites Mal wird der Bypassmodus derart eingestellt, dass sich zwischen dem Blut und der (verbleibenden) Dialysierflüssigkeit kein Konzentrationsgleichgewicht einstellt. Bei jedem Bypassmodus wird eine Größe der verbrauchten Dialysierflüssigkeit, insbesondere der Anteil an harnstoffpflichtigen Stoffen, erfasst. Aus dem Verhältnis der erfassten Größen in der Dialysierflüssigkeit nach Beendigung des Bypassmodus kann die Rezirkulationsrate der Blutbehandlung ermittelt werden. Es ist selbstverständlich auch denkbar zuerst einen kurzen Bypass und anschließend einen langen Bypass durchzuführen.

Kurzbeschreibung der Figuren

Fig. 1 ist eine Darstellung zur Veranschaulichung eines Systemaufbaus gemäß einer ersten Ausführungsform der vorliegenden Offenbarung;

Fig. 2 ist eine Darstellung zur Veranschaulichung eines Systemaufbaus einer Sensoreinheit;

Fig. 3 ist eine Darstellung einer Dialysemaschine mit einem Dialysator und einer separaten offenbarungsgemäßen Vorrichtung;

Fig. 4 ist eine Darstellung einer zweiten Ausführungsform mit zwei Messvorrichtungen;

Fig. 5 ist eine Darstellung einer dritten Ausführungsform mit lediglich einer Messvorrichtung;

Fig. 6 ist ein Ablaufdiagramm eines offenbarungsgemäßen Verfahrens;

Fig. 7 ist eine Darstellung gemäß einer vierten Ausführungsform der vorliegenden Offenbarung mit einer kombinierten Sensor-, Elektronik- und Auswerteeinheit und einer Bedieneinheit;

Fig. 8 ist eine Darstellung gemäß einer fünften Ausführungsform der vorliegenden Offenbarung mit einer Sensoreinheit und einer kombinierten Elektronik-, Auswerte- und Bedieneinheit;

Fig. 9 ist eine Darstellung gemäß einer sechsten Ausführungsform der vorliegenden Offenbarung mit einer kombinierten Sensor-, Elektronik-, Auswerte- und Bedieneinheit;

Detaillierte Beschreibung der Ausführungsformen

Nachstehend werden Ausführungsformen der vorliegenden Offenbarung auf der Basis der zugehörigen Figuren beschrieben.

Erste Ausführungsform

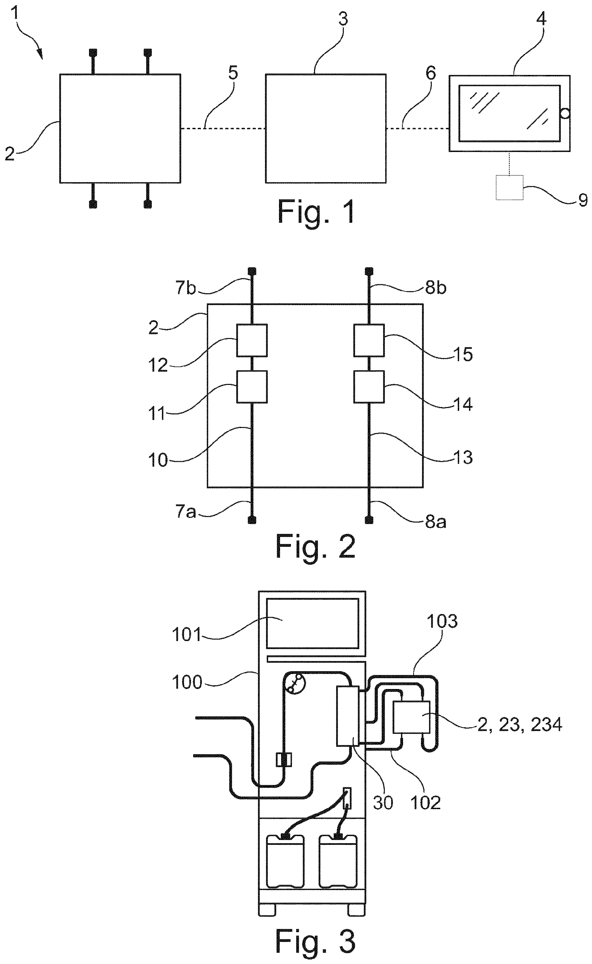

Fig. 1 zeigt eine Vorrichtung zur Elektrolytbilanzierung, im folgenden auch Elektrolytbilanzierungsmodul (EBM) 1 genannt. Das EBM 1 weist eine Sensoreinheit 2, eine Elektronik- und Auswerteeinheit 3 und eine Bedieneinheit 4 mit einem Touchscreen auf.

Zwischen der Sensoreinheit 2 und der Elektronik- und Auswerteeinheit 3 besteht eine elektrische Verbindung 5. Zwischen der Elektronik- und Auswerteeinheit 3 und der Bedieneinheit 4 besteht ebenfalls eine Verbindung 6, die entweder ebenfalls kabelgebunden ist oder aber bevorzugt kabellos ausgebildet ist, beispielsweise über Bluetooth oder WLAN. Die Spannungsversorgung des EBMs 1 erfolgt über einen in der Elektronik- und Auswerteeinheit 3 verbauten Akku oder über eine externe Spannungsquelle 9.

Fig. 2 zeit die Sensoreinheit 2. Über den Anschluss 7a kann eine Dialysierflüssigkeit durch eine Zuführleitung 10 in die Sensoreinheit 2 fließen und diese wieder über einen Anschluss 7b verlassen. Eine Messvorrichtung 11 misst dabei eine chemische und/oder physikalische Größe der Dialysierflüssigkeit. Vorzugsweise handelt es sich dabei um die temperaturkompensierte Leitfähigkeit der Flüssigkeit. Als Messvorrichtung 11 sind auch ionenselektive Elektroden oder optische Vorrichtungen zur Bestimmung von Ionenkonzentrationen (beispielsweise Laserinduzierte Plasmaspektroskopie) denkbar. Die Messvorrichtung 11 kann entweder invasiv messen, also mit Kontakt zu der Dialysierflüssigkeit, oder nicht- invasiv. Letzteres kann bspw. mittels eines Gradiometers (wie in DE 10 2014 116 415 A1 offenbart) oder optisch geschehen. Die Messvorrichtung 12 ist optional. Es kann sich hierbei um einen Flussdetektor (beispielsweise auf Ultraschallbasis) handeln, der eine Flussrate der Dialysierflüssigkeit in der Zuführleitung 10 bestimmt. Alternativ kann er auch derart ausgebildet sein, dass er lediglich feststellt, ob sich die Dialysierflüssigkeit bewegt oder steht.

Über den Anschluss 8a strömt verbrauchte Dialysierflüssigkeit, in die Abführleitung 13 der Sensoreinheit 2. Über den Anschluss 8b verlässt die verbrauchte Dialysierflüssigkeit die Sensoreinheit 2 wieder. Die Messvorrichtung 14 misst eine chemische und/oder eine physikalische Größe der verbrauchten Dialysierflüssigkeit. Auch hier handelt es sich bevorzugt um die temperaturkompensierte Leitfähigkeit der Flüssigkeit oder um eine Ionenkonzentration. Die Messvorrichtung 15 misst ebenfalls eine chemische und/oder physikalische Größe. Vorzugsweise handelt es sich um einen optischen Sensor zur Bestimmung einer Absorptionseigenschaft der verbrauchten Dialysierflüssigkeit, beispielsweise die Extinktion der verbrauchten Dialysierflüssigkeit. Alternativ ist auch ein enzymatischer oder ein anderer elektrochemischer Sensor denkbar. Die Messvorrichtung 15 ist optional. Die Anschlüsse 7a, 7b, 8a, 8b sind vorzugsweise Schnellkupplungen.

Die Elektronik- und Auswerteeinheit 3 umfasst Schaltungen zur Generierung der Anregungsspannungen für die Leitfähigkeitssonden, Schaltungen zur Verstärkung und Digitalisierung analoger Messsignale und einen Mikrocontroller zur Auswertung der Messsignale.

Fig. 3 zeigt, wie die Sensoreinheit 2 an einer Dialysemaschine 100 angebracht sein kann. Die Dialysemaschine 100 weist einen Dialysator 30, eine Bedienoberfläche 101, eine Dialysierflüssigkeitsleitung 102 und eine Leitung für verbrauchte Dialysierflüssigkeit 103 auf. Aus Gründen der Übersichtlichkeit sind die restlichen Komponenten des EBMs 1 hier nicht dargestellt. Die Sensoreinheit 2 wird vor der Durchführung der Dialysebehandlung an die Dialysemaschine 100 angeschlossen. Über die Dialysierflüssigkeitsleitung 102 strömt Dialysierflüssigkeit aus der Dialysemaschine 100 durch die Sensoreinheit 2 in den Dialysator 30. Hier kommt es zum Stoffaustausch zwischen der Dialysierflüssigkeit und dem Blut des (nicht dargestellten) Patienten. Die Dialysierflüssigkeit nimmt überschüssiges Wasser und Schadstoffe aus dem Blut auf und gibt ggf. andere Stoffe, insbesondere Hydrogencarbonat und/ oder andere Elektrolyte bzw. Nichtelektrolyte, an das Blut ab. Über die Leitung für verbrauchte Dialysierflüssigkeit 103 strömt die verbrauchte Dialysierflüssigkeit aus dem Dialysator 30 in die Einheit 2, wo es von der Messvorrichtung 14 und gegebenenfalls der Messvorrichtung 15 vermessen werden kann.

Die Sensoreinheit 2 mit den Anschlüssen 7a, 7b, 8a, 8b wird vor der Dialysebehandlung an die Dialysemaschine 100 angebracht. Die Sensoreinheit 2 wird an Schläuche/ Leitungen der Dialysemaschine 100 angebracht, die nach außen aus der Dialysemaschine 100 herausstehen. Die Montage des EBMs 1 wird dabei von geschultem Personal, wie einem Techniker oder ähnlichem, vorgenommen. Die Leitungen werden aufgetrennt und auf die Enden der Leitungen werden Verbindungsstücke aufgesetzt. Die Verbindungstücke passen mit den Anschlüssen 7a, 7b, 8a, 8b zusammen und bilden Schnellkupplungen.

Es ist offensichtlich, dass die Sensoreinheit 2 nicht nur an die Leitungen 102, 103 angebracht werden kann, sondern auch direkt an die Anschlüsse der Dialysemaschine. Das heißt, die Leitungen 102, 103 entfallen und die Sensoreinheit 2 wird direkt an der Dialysemaschine angeschlossen. Zum Anschließen können die Anschlüsse verwendet werden, die für die Leitungen 102, 103 vorgesehen sind.

Zweite Ausführungsform

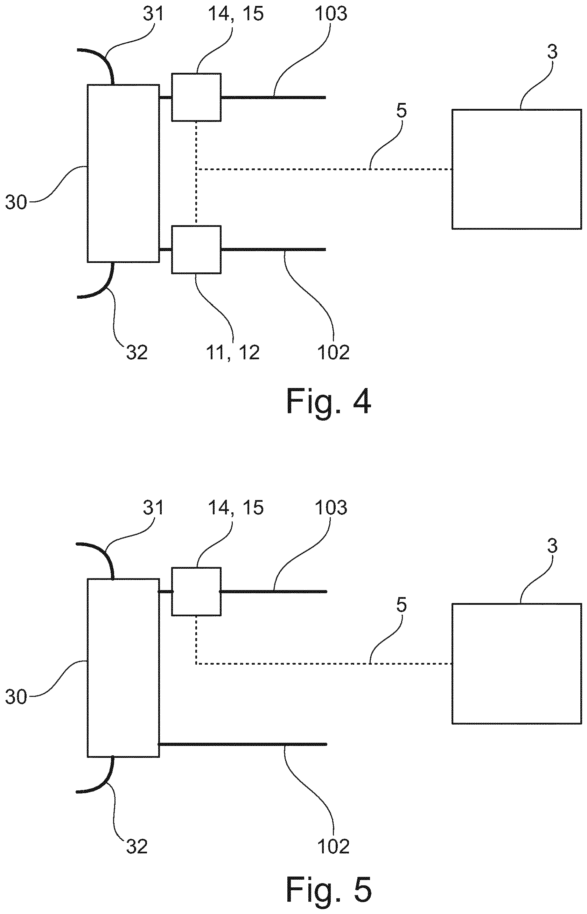

Anstatt die Messvorrichtungen 11, 12, 14, 15 in einer gemeinsamen Sensoreinheit 2 unterzubringen, können diese auch getrennt voneinander vorliegen. Fig. 4 zeigt exemplarisch eine zweite Ausführungsform, bei der die Messvorrichtung 11 und/ oder die Messvorrichtung 12 an der Dialysierflüssigkeit führenden Dialysierflüssigkeitsleitung 102 angebracht sind/ist. Dies kann durch Auftrennen der Dialysierflüssigkeitsleitung 102 und Einbringen der Messvorrichtung erfolgen. Handelt es sich bei den Messvorrichtungen 11, 12 um nicht-invasiv arbeitende Messvorrichtungen, muss die Leitung nicht aufgetrennt werden. Die Messvorrichtungen 11, 12 können stattdessen von außen auf die Leitung angebracht werden. Gleiches gilt für die Messvorrichtungen 14, 15 und die Leitung für verbrauchte Dialysierflüssigkeit 103, welche verbrauchte Dialysierflüssigkeit aus dem Dialysator 30 führt. Der Dialysator 30 weist einen Bluteingang 31 und einen Blutausgang 32 auf.

Dritte Ausführungsform

Fig. 5 zeigt eine dritte Ausführungsform der Offenbarung. Da für die Elektrolytbilanzierung insbesondere die chemischen und/oder physikalischen Größen in der verbrauchten Dialysierflüssigkeit der Leitung 103 von Interesse sind, reicht es in einem einfachsten Fall aus, lediglich in der Leitung für verbrauchte Dialysierflüssigkeit 103 eine Messvorrichtung 14 mit der optionalen Messvorrichtung 15 vorzusehen.

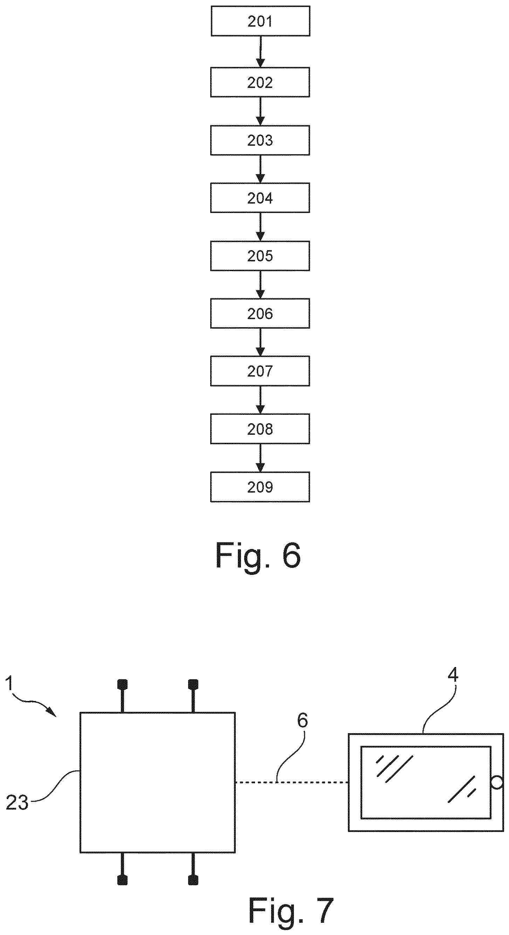

In DE 10 2017 116 097 A1 wird ein Verfahren zur Durchführung einer isonaträmischen Dialyse, also zur Bilanzierung von Natrium, beschrieben. Dieses Verfahren kann wie folgt mit dem separaten EBM 1 durchgeführt werden. Fig. 6 zeigt das Verfahren.

Schritt 201: Zuerst wird mit dem Betätigen einer Schaltfläche auf dem Touchscreen der Bedieneinheit 4 des EBMs 1 eine Bestimmung der Plasma-Natrium-Konzentration bzw. einer Größe, die mit der Plasma-Natrium-Konzentration korreliert, initialisiert. Um den Einfluss möglicher Elektrolytverschiebungen aufgrund von Diffusionsprozessen in dem Dialysator auf ein Minimum zu reduzieren, sollte die Bestimmung der initialen Plasma-Natrium-Konzentration mithilfe des in DE 10 2017 116 097 A1 in seinen Grundzügen beschriebenen Verfahrens zu Beginn einer Dialysebehandlung erfolgen. Beispielsweise sollte dies innerhalb der ersten 15 Minuten geschehen.

Schritt 202: Im nächsten Schritt werden für eine definierte Zeit von bspw. 10 Sekunden Messwerte an dem Dialysatorausgang 103 und ggf. an dem Eingang 102 des Dialysators 30 aufgenommen.

Dabei wird eine Größe erfasst, die mit der Plasma-Natrium-Konzentration im Blut eines Patienten korreliert, indem bei einem Dialysator die Zufuhr von Dialysierflüssigkeit bei einer laufenden Dialysebehandlung kurzzeitig abgeschaltet wird. Die verbleibende Dialysierflüssigkeit hat deutlich weniger Volumen als ein Blutstrom. Dadurch nimmt die verbleibende Dialyseflüssigkeit die Natrium-Konzentration des Blutes bis zur Sättigung auf. Durch Messung der Natrium-Konzentration der verbrauchten Dialysierflüssigkeit am Ausgang des Dialysators kann auf die Plasma-Natrium-Konzentration des Blutes geschlossen werden, ohne im Blutkreislauf der Dialysemaschine messen zu müssen. Die Bestimmung der Größe, die mit der Plasma-Natrium-Konzentration im Blut eines Patienten korreliert, muss manuell von dem Benutzer an der Dialysemaschine initiiert werden.

Die Erfassung der Größe soll am Anfang der Dialysebehandlung erfolgen, um die ursprüngliche Plasma-Natrium-Konzentration im Blut des Patienten zu ermitteln. Für den Start der Erfassung sind mehrere Optionen denkbar. Zum einen kann das Verfahren durch den Benutzer beim Start der Dialysebehandlung gestartet werden. Eine andere Option ist, durch die Messvorrichtung 15 die Konzentration an harnpflichtigen Stoffen in der verbrauchten Dialysierflüssigkeit zu messen. Vor dem Start der Blutbehandlung ist die Konzentration an harnpflichtigen Stoffen annähernd null. Da die Konzentration in der verbrauchten Dialysierflüssigkeit beim Beginn der Dialysebehandlung stark ansteigt, ist es möglich den Beginn der Dialysebehandlung durch die Messvorrichtung 15 zu erfassen. Es ist sowohl denkbar, dass das EBM 1 die Messung automatisch nach einer definierten Zeitspanne nach dem Beginn der Dialysebehandlung startet, als auch, dass auf der Bedieneinheit 4 ein optisches Signal ausgegeben wird, dass verdeutlicht, dass die Messung gestartet werden kann. Es ist offensichtlich, dass das Signal auch akustisch sein kann. Es ist denkbar, dass das Signal nach Ablauf einer bestimmten Zeitspanne, beispielsweise 15 Minuten, erlöscht und damit signalisiert, dass eine Erfassung der Plasma-Natrium-Konzentration nicht mehr sinnvoll ist.

Es ist auch denkbar, dass die Messung der Plasma-Natrium-Konzentration zu einem beliebigen Zeitpunkt während der Dialysebehandlung gestartet werden kann. Wenn die Plasma-Natrium-Konzentration nicht zu Beginn, sondern während der Dialysebehandlung gemessen wird, wird dadurch nicht die ursprüngliche Plasma-Natrium-Konzentration im Blut des Patienten gemessen, aber es kann überprüft werden, ob die Plasma-Natrium-Konzentration während der Behandlung konstant bleibt.

Schritt 203: Anschließend folgt die Aufforderung an der Bedieneinheit 4, die Dialysemaschine 100 in den Bypassmodus zu schalten.

In dem Bypassmodus fließt keine Dialysierflüssigkeit durch den Dialysator. Die Dialysierflüssigkeit wird durch ein Bypassventil abgeleitet, umgeht den Dialysator und wird zu einem Abfluss geleitet.

Schritt 204: Über die Bedienoberfläche 101 der Dialysemaschine 100 wird manuell in den Bypassmodus geschaltet. Der Bypassmodus wird von dem optionalen Sensor 12 erkannt. Es ist ebenfalls möglich, statt in den Bypassmodus in die sogenannte sequentielle Phase umzuschalten. Hierbei wird die Reinigung des Blutes über Konvektion bzw. Ultrafiltration fortgesetzt.

Schritt 205: Nach Ablauf einer definierten Dauer von bspw. 90 Sekunden erscheint auf der Bedieneinheit 4 die Aufforderung, die Dialysemaschine 100 wieder in den Hauptschluss zu schalten.

Der Hauptschluss oder Normalbetriebsmodus ist der Modus der Dialyse, bei dem frische Dialysierflüssigkeit in einen Dialysator geleitet wird und verbrauchte Dialysierflüssigkeit aus dem Ausgang des Dialysators austritt.

Schritt 206: Der Bypassmodus der Dialysemaschine 100 wird manuell deaktiviert. Die Beendigung des Bypassmodus wird von dem optionalen Sensor 12 erkannt.

Schritt 207: Die Messvorrichtungen 14 und ggf. 15 starten die Messwertaufnahme für eine Dauer von bspw. 25 Sekunden.

Schritt 208: Die aufgenommenen Messwerte werden von der Elektronik- und Auswerteeinheit 3 analysiert und es wird darauf basierend eine empfohlene Leitfähigkeit der Dialysierflüssigkeit bzw. Natrium-Konzentration in der Dialysierflüssigkeit auf der Bedieneinheit 4 ausgegeben, die im Falle einer gewollten isonaträmischen Dialyse dafür sorgt, dass die Plasma-Natrium-Konzentration des Patienten im Wesentlichen im Laufe einer Dialysebehandlung konstant bleibt.

Schritt 209: Auf der Bedienoberfläche 101 der Dialysemaschine 100 wird die empfohlene Soll-Leitfähigkeit bzw. Soll-Konzentration manuell eingegeben, woraufhin die Maschine mit bekannten Mitteln und Methoden die Anpassung der Dialysierflüssigkeitszusammensetzung vornimmt.

Da während des Bypasses kein Fluss durch die Zuführleitung 10 und die Abführleitung 13 bzw. Dialysierflüssigkeitsleitung 102 und die Leitung für verbrauchte Dialysierflüssigkeit 103 vorhanden ist, kann der Bypassmodus von dem Flussdetektor 12 erkannt werden. Alternativ kann der Bypassmodus auch von einem Hallsensor erkannt werden, welcher den Schaltzustand des üblicherweise elektromagnetisch betriebenen Bypassventils innerhalb der Dialysemaschine 100 erfasst.

Alternativ kann Beginn und Ende des Bypassmodus manuell an dem Touchscreen der Bedieneinheit 4 bestätigt werden, sodass die Messvorrichtung 12 komplett entfallen kann.

Wie bereits erwähnt, handelt es sich bei der Messvorrichtung 14 vorzugsweise um eine temperaturkompensierende Leitfähigkeitssonde. Obwohl Natrium und seine Anionen, insbesondere Chlorid und Hydrogencarbonat, die Stoffe in Flüssigkeiten wie Dialysierflüssigkeit oder Plasmawasser sind, die am stärksten zur Leitfähigkeit beitragen, wird die Leitfähigkeitsmessung von weiteren Stoffen beeinflusst, sodass eine einfache Umrechnung zwischen Leitfähigkeit und Natrium-Konzentration nicht mehr möglich ist. Beispielsweise können erhöhte Kalium-Werte die Leitfähigkeit erhöhen. Es gibt aber auch Stoffe, die per se nicht leitfähig sind und dennoch die Leitfähigkeit beeinträchtigen können, da sie die Beweglichkeit der leitenden Ionen herabsetzen. Dieser Effekt wird von Toxinen und anderen harnpflichtigen Substanzen verursacht.

Insbesondere zu Beginn einer Dialysebehandlung passieren viele dieser Stoffe die semipermeable Membran in dem Dialysator 30 und gelangen so auf die Seite der Dialysierflüssigkeit. Die Messvorrichtung 14 misst deshalb eine Leitfähigkeit, die aufgrund dieser Substanzen herabgesetzt wird. Würde nun aber die Proportionierungseinheit der Dialysemaschine 100 exakt diese Leitfähigkeit anmischen, würde dem Patienten dadurch im Laufe der Dialysebehandlung unerwünscht Natrium entzogen. Um diesem Effekt entgegenzuwirken, ist es notwendig, die Leitfähigkeit an der Messvorrichtung 14 zu korrigieren. Eine starre Korrektur der Leitfähigkeit durch Addieren eines festen Betrags ist allerdings nachteilhaft, da die Belastung mit Toxinen von Patient zu Patient und von Behandlung zu Behandlung unterschiedlich ausfallen kann. Die bevorzugte Ausführungsform der Erfindung sieht deshalb vor, an dem Dialysatorausgang neben der Messvorrichtung 14 eine weitere Messvorrichtung 15 anzubringen, welche eine Absorptionseigenschaft in der verbrauchten Dialysierflüssigkeit bestimmt.

Zur Korrektur der dialysierflüssigkeitsseitigen Leitfähigkeit kann die Absorptionseigenschaft der verbrauchten Dialysierflüssigkeit als ein Maß für die Toxinbelastung herangezogen werden. Eine mögliche Absorptionseigenschaft ist die Extinktion.

Die Leitfähigkeit wird korrigiert, indem die von Messvorrichtung 14 gemessene Leitfähigkeit $C_{DO}$ und die von Messvorrichtung 15 gemessene Extinktion A mathematisch miteinander kombiniert werden:

$$C_{DO,corr} = f(C_{DO}, A)$$

Im einfachsten Fall bietet sich eine lineare Gleichung an. Aber auch Polynome höheren Grades oder eine Gleichung, die mithilfe eines künstlichen neuronalen Netzes ermittelt wurde, sind denkbar. Eine alternative Möglichkeit ist die Verwendung einer Lookup-Tabelle.

Soll beispielsweise eine null-diffusive Natrium-bilanzierte Dialyse durchgeführt werden, so wird die Dialysierflüssigkeit von der Proportionierungseinheit derart zusammengemischt, dass ihre Leitfähigkeit $C_{DI}$ der korrigierten Leitfähigkeit der verbrauchten Dialysierflüssigkeit $C_{DO,corr}$ entspricht:

$$C_{DI} = C_{DO,corr}$$

Sind beide Leitfähigkeiten gleich, so findet in dem Dialysator kein diffusiver Austausch von Natriumionen zwischen Blutseite und der Seite der Dialysierflüssigkeit statt. Die Leitfähigkeit $C_{DI}$ bzw. $C_{DO,corr}$ entspricht somit auch der Plasmawasserleitfähigkeit $C_{BI}$, die proportional zur Plasma-Natrium-Konzentration in dem Patientenblut ist. Es ist daher auch möglich, nach anfänglicher Ermittlung der Plasma-Natrium-Konzentration die Dialysierflüssigkeit derart einzustellen, dass dem Patienten eine definierte Menge Natrium entzogen oder verabreicht werden kann. Der Natriumfluss $\Delta J$ berechnet sich dabei zu:

$$\Delta J = k \cdot Q_D \cdot (C_{DI} - C_{DO,corr}) + k \cdot Q_{UF} \cdot C_{DO,corr}$$

mit dem Dialysierflüssigkeitsfluss $Q_D$, der Ultrafiltrationsrate $Q_{UF}$ und einem Faktor $k$, mit welchem die Leitfähigkeit in eine Natriumkonzentration umgerechnet wird, wobei auch hier wieder komplexere Umrechnungen zum Einsatz kommen können.

Darüber hinaus ermöglicht das Verfahren die Bestimmung der absolut entzogenen Menge an Natrium. Entweder anhand vorheriger Gleichung unter Berücksichtigung der Therapiedauer oder: Bei bekanntem Ultrafiltrationsvolumen und der Kenntnis der Plasma-Natrium-Konzentration im Verlauf der Dialysebehandlung entspricht die entzogene Menge an Natrium dem Produkt aus Ultrafiltrationsvolumen und Plasma-Natrium-Konzentration.

Um das Verfahren für das medizinische Personal und/oder den Anwender transparent zu gestalten, sieht es die Erfindung vor, Daten der Messvorrichtungen auf dem Bildschirm der Bedieneinheit 4 anzeigen zu lassen. Das betrifft insbesondere Leitfähigkeiten, Konzentrationen, Extinktionen, gegebenenfalls pH-Werte, und Temperaturen.

Nach der Durchführung der Dialysebehandlung bleibt das EBM 1 vorzugsweise auch während der anschließenden Desinfektion an der Dialysemaschine angeschlossen, um ebenfalls desinfiziert und entkalkt werden zu können.

Ein weiteres Merkmal der vorliegenden Erfindung sieht vor, erhobene Daten auf einer Patientenkarte und/oder in einem Datenmanagementsystem abzuspeichern und somit für weitere Analysen zur Verfügung zu stellen. Hierfür weist das EBM 1 eine entsprechende Aufnahmevorrichtung mit Lese- und Schreibefunktion der Patientenkarte auf. Das Übermitteln der Daten auf das Datenmanagementsystem, welches häufig in Dialysekliniken oder ähnlichen Einrichtungen vorzufinden ist, kann kabellos (z.B. über WLAN) oder kabelgebunden (z.B. über LAN) geschehen.

Darüber hinaus ist es gemäß der Erfindung möglich, alternativ zur Bedieneinheit mit Touchscreen 4 ein Smartphone, ein Tablet oder ein ähnliches Gerät mit einer Eingabevorrichtung, z.B. Touchscreen, zur Bedienung und computerähnlichen Funktionalitäten und Konnektivitäten mit entsprechender Applikation zu verwenden. Dies ist insbesondere im Bereich der Heimdialyse vorteilhaft, wenn die Dialysepatienten auf ihre eigenen Geräte zurückgreifen können.

Vierte Ausführungsform

Fig. 7 zeigt eine vierte Ausführungsform der Erfindung. Dabei müssen die Sensoreinheit 2 und die Elektronik- und Auswerteeinheit 3 nicht in zwei unterschiedlichen Einheiten verbaut sein. Die Sensoreinheit 2 und die Elektronik- und Auswerteeinheit 3 können auch in eine gemeinsame Sensor-, Elektronik- und Auswerteeinheit 23 zusammengefasst sein. Die Sensor-, Elektronik- und Auswerteeinheit 23 ist mit der Bedieneinheit 4 über die Verbindung 6 verbunden.

Fünfte Ausführungsform

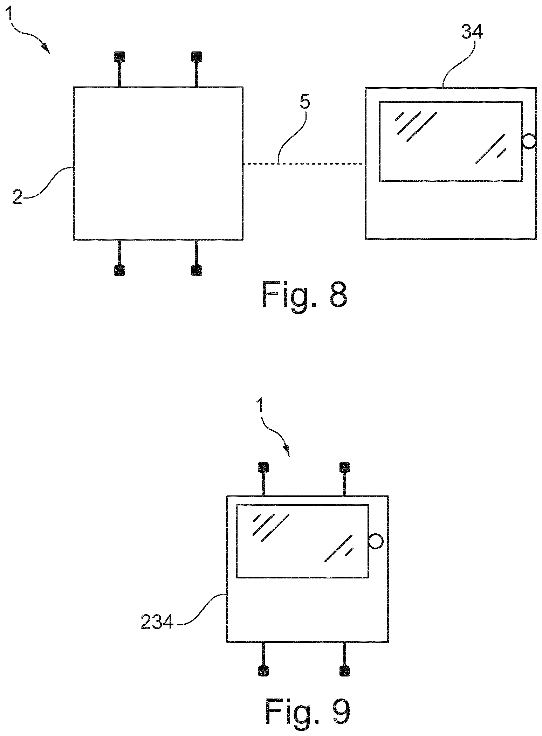

Fig. 8 zeigt eine fünfte Ausführungsform der Erfindung. Dabei müssen die Elektronik- und Auswerteeinheit 3 und die Bedieneinheit 4 nicht in zwei unterschiedlichen Einheiten verbaut sein. Die Elektronik- und Auswerteeinheit 3 und die Bedieneinheit 4 können auch in eine gemeinsame Elektronik-, Auswerte- und Bedieneinheit 34 zusammengefasst sein. Die Elektronik-, Auswerte- und Bedieneinheit 34 ist mit der Sensoreinheit 2 über die Verbindung 5 verbunden.

Sechste Ausführungsform

Fig. 9 zeigt eine noch kompaktere Ausführungsform der Offenbarung. In einer sechsten Ausführungsform sind die Sensoreinheit 2, die Elektronik- und Auswerteeinheit 3 und die Bedieneinheit 4 zu einer gemeinsamen Einheit 234 zusammengefasst.

**Patentansprüche**

1. Vorrichtung zur Elektrolytbilanzierung zur Nachrüstung einer extrakorporalen Blutbehandlungsmaschine (100), insbesondere einer Hämodialysemaschine, aufweisend:

   - eine Sensoreinheit (2), mit
   - mindestens einer ersten Messvorrichtung (14) zum Erfassen eines Natriumgehalts einer verbrauchten Dialysierflüssigkeit,

- einem Anschlussabschnitt oder -teil (7a, 8a) zum vorzugsweise adaptiven Anschließen der Sensoreinheit (2) an den Fluidkreis einer Blutbehandlungsmaschine (100), derart, dass die erste Messvorrichtung (14) zum Erfassen des Natriumgehalts geeignet ist, und

- eine unabhängig von der extrakorporalen Blutbehandlungsmaschine (100) arbeitende Elektronik- und Auswerteeinheit (3), die den von der ersten Messvorrichtung (14) erfassten Natriumgehalt analysiert und in Abhängigkeit des erfassten Natriumgehalts eine Empfehlung für einen Natriumgehalt einer frischen Dialysierflüssigkeit ermittelt und

- eine, vorzugsweise kombinierte Eingabe-/Ausgabeschnittstelle (4), die dafür ausgebildet ist, einem Benutzer die von der Elektronik- und Auswerteeinheit (3) ermittelte Empfehlung für den Natriumgehalt der frischen Dialysierflüssigkeit auszugeben, und

- eine unabhängig von der extrakorporalen Blutbehandlungsmaschine (100) ausgebildete Energieversorgung (9).

2. Vorrichtung nach Anspruch 1, **gekennzeichnet durch** eine maschinenlesbare Speichereinheit, auf der ein Programm mit den folgenden Schritten abgespeichert ist:

- Initialisieren der Erfassung einer mit der Plasma-Natrium-Konzentration im Blut des Patienten korrelierten Größe durch die erste Messvorrichtung (14) durch den Benutzer/Bediener über die Bedieneinheit (4);
- Erfassen des Natriumgehalts der verbrauchten Dialysierflüssigkeit durch die erste Messvorrichtung (14);
- Anweisen des Benutzers/Bedieners über die Bedieneinheit (4), die Blutbehandlungsmaschine (100) in einen Bypassmodus zu schalten;
- Informieren der Vorrichtung über den Bypassmodus beispielsweise durch selbständiges sensorisches Erkennen oder durch eine händische Eingabe an der Bedieneinheit (4);
- Anweisen des Benutzers/Bedieners über die Bedieneinheit (4), die Blutbehandlungsmaschine (100) in den Normalbetriebsmodus zu schalten;
- Informieren der Vorrichtung über den Normalbetriebsmodus beispielsweise durch selbständiges sensorisches Erkennen oder durch händische Eingabe an der Bedieneinheit (4);
- Erfassen des Natriumgehalts der verbrauchten Dialysierflüssigkeit durch die erste Messvorrichtung (14);
- Analyse des erfassten Natriumgehalts der verbrauchten Dialysierflüssigkeit durch die Elektronik- und Auswerteeinheit (3) und Ausgabe der Empfehlung eines Natriumgehalts der frischen Dialysierflüssigkeit in Abhängigkeit des erfassten Natriumgehalts in der verbrauchten Dialysierflüssigkeit über die Bedieneinheit (4);
- Anweisen des Benutzers/Bedieners über die Bedieneinheit (4), den empfohlenen Natriumgehalt an der Blutbehandlungsmaschine (100) einzustellen.

3. Vorrichtung nach Anspruch 1 oder 2, **gekennzeichnet durch** eine zweite Messvorrichtung (11), die in einer Zuführleitung (10) vorgesehen ist und den Natriumgehalt der frischen Dialysierflüssigkeit erfasst.

4. Vorrichtung nach Anspruch 3, **dadurch gekennzeichnet, dass** die erste Messvorrichtung (14) und die zweite Messvorrichtung (11) sowohl invasiv als auch nicht-invasiv messen können.

5. Vorrichtung nach Anspruch 4, **dadurch gekennzeichnet, dass** die erste Messvorrichtung (14) und die zweite Messvorrichtung (11) eine temperaturkompensierte Leitfähigkeit der Dialysierflüssigkeit bestimmen.

6. Vorrichtung nach Anspruch 3 oder 4, **dadurch gekennzeichnet, dass** die erste Messvorrichtung (14) und die zweite Messvorrichtung (11) eine Ionenkonzentration in der Dialysierflüssigkeit bestimmen.

7. Vorrichtung nach einem der Ansprüche 1 bis 6, **dadurch gekennzeichnet, dass** die Vorrichtung drei Ventile aufweist, durch die der Bypassmodus an der Vorrichtung eingestellt wird, wobei in dem Bypassmodus zumindest ein Eingangsventil geschlossen und zumindest ein Ventil geöffnet ist, um die Dialysierflüssigkeit um einen Dialysator zu lenken.

8. Vorrichtung nach einem der Ansprüche 1 bis 7, **dadurch gekennzeichnet, dass** die Empfehlung für den Natriumgehalt der Dialysierflüssigkeit von der Elektronik- und Auswerteeinheit (3) derart gewählt wird, dass eine Plasma-Natrium-Konzentration im Blut eines Patienten über eine Dauer der Blutbehandlung im Wesentlichen gleichbleibt.

9. Vorrichtung nach einem der Ansprüche 1 bis 8, **gekennzeichnet durch** eine weitere Messvorrichtung (15), die eine chemische oder physikalische Größe, vorzugsweise eine Absorptionseigenschaft wie eine Extinktion, insbesondere einen Harnstoffgehalt, der verbrauchten Dialysierflüssigkeit in der Abführleitung (13) bestimmt.

**10.** Vorrichtung nach Anspruch 9, **dadurch gekennzeichnet, dass** die Messvorrichtungen (11, 14, 15) einzeln direkt an den jeweiligen Leitungen (10, 13) angebracht werden.

**11.** Vorrichtung nach einem der Ansprüche 1 bis 10, **dadurch gekennzeichnet, dass** die Sensoreinheit (2), die Elektronik- und Auswerteeinheit (3) und die Bedieneinheit (4) in einer Einheit (234) zusammengefasst sind.

**12.** Vorrichtung nach einem der Ansprüche 1 bis 11, **dadurch gekennzeichnet, dass** die Vorrichtung bei der Messung der temperaturkompensierenden Leitfähigkeit eine Auswirkung von Stoffen, die nicht Natrium sind, auf die gemessene Leitfähigkeit erfasst und die gemessene Leitfähigkeit gemäß einer Existenz der anderen Stoffe korrigiert.

**13.** Verfahren zur Anbringung der Vorrichtung nach den Ansprüchen 1 bis 12 an der Dialysemaschine aufweisend:

- Anbringen der ersten Messvorrichtung (14) an der Abführleitung (13) der Dialysemaschine,
- gegebenenfalls Anbringen einer optionalen vierten Messvorrichtung (15) an der Abführleitung (13) der Dialysemaschine,
- gegebenenfalls Anbringen der zweiten Messvorrichtung (11) an der Zuführleitung (10) der Dialysemaschine,
- gegebenenfalls Anbringen einer optionalen dritten Messvorrichtung (12) an der Zuführleitung (10) der Dialysemaschine.

**14.** Verfahren nach Anspruch 13, **dadurch gekennzeichnet, dass** die Sensoreinheit (2) invasiv an der Leitung (102, 103) angebracht wird, indem:

- die Leitung in zwei Teile getrennt wird,
- die Sensoreinheit (2) mit dem ersten Teil verbunden wird, und
- die Sensoreinheit (2) mit dem zweiten Teil verbunden wird.


**Claims**

**1.** A device for electrolyte balancing for retrofitting an extracorporeal blood treatment machine (100), in particular a hemodialysis machine, comprising:

- a sensor unit (2), having
- at least a first measuring device (14) for detecting a sodium content of a used dialysis fluid,
- a connection portion or part (7a, 8a) for preferably adaptively connecting the sensor unit (2) to the fluid circuit of a blood treatment machine (100), such that the first measuring device (14) is suitable for detecting the sodium content, and
- an electronics and evaluation unit (3) operating independently of the extracorporeal blood treatment machine (100), which analyzes the sodium content detected by the first measuring device (14) and determines a recommendation for a sodium content of a fresh dialysis fluid in dependence on the detected sodium content, and
- a preferably combined input/output interface (4) which is configured to output to a user the recommendation for the sodium content of the fresh dialysis fluid determined by the electronics and evaluation unit (3), and
- a power supply (9) configured independently of the extracorporeal blood treatment machine (100).

**2.** The device according to claim 1, **characterized by** a machine-readable memory unit on which a program comprising the following steps is stored:

- initializing the detection of a quantity correlated with the plasma-sodium concentration in the patient's blood by the first measuring device (14) by the user/operator via the operating unit (4);
- detecting the sodium content of the used dialysis fluid by the first measuring device (14);
- instructing the user/operator via the operating unit (4) to switch the blood treatment machine (100) into a bypass mode;
- informing the device about the bypass mode, for example by automatic sensory recognition or by manual input at the operating unit (4);
- instructing the user/operator via the operating unit (4) to switch the blood treatment machine (100) to normal operating mode;
- informing the device about the normal operating mode, for example by automatic sensory recognition or by manual input at the operating unit (4);

- detecting the sodium content of the used dialysis fluid by the first measuring device (14);
- analyzing the detected sodium content of the used dialysis fluid by the electronics and evaluation unit (3) and outputting the recommendation of a sodium content of the fresh dialysis fluid depending on the detected sodium content in the used dialysis fluid via the operating unit (4);
- instructing the user/operator via the operating unit (4) to set the recommended sodium content on the blood treatment machine (100).

3. The device according to claim 1 or 2, **characterized by** a second measuring device (11) provided in a supply line (10) and detecting the sodium content of the fresh dialysis fluid.

4. The device according to claim 3, **characterized in that** the first measuring device (14) and the second measuring device (11) can measure both invasively and non-invasively.

5. The device according to claim 4, **characterized in that** the first measuring device (14) and the second measuring device (11) determine a temperature-compensated conductivity of the dialysis fluid.

6. The device according to claim 3 or 4, **characterized in that** the first measuring device (14) and the second measuring device (11) determine an ion concentration in the dialysis fluid.

7. The device according to one of claims 1 to 6, **characterized in that** the device comprises three valves by which the bypass mode is set on the device, wherein in the bypass mode, at least one input valve is closed and at least one valve is open in order to direct the dialysis fluid around a dialyzer.

8. The device according to one of claims 1 to 7, **characterized in that** the recommendation for the sodium content of the dialysis fluid is selected by the electronics and evaluation unit (3) such that a plasma sodium concentration in the blood of a patient remains substantially constant over a duration of the blood treatment.

9. The device according to one of claims 1 to 8, **characterized by** a further measuring device (15) which determines a chemical or physical quantity, preferably an absorption property such as an extinction, in particular a urea content, of the used dialysis fluid in the discharge line (13).

10. The device according to claim 9, **characterized in that** the measuring devices (11, 14, 15) are individually attached directly to the respective lines (10, 13).

11. The device according to one of claims 1 to 10, **characterized in that** the sensor unit (2), the electronics and evaluation unit (3) and the operating unit (4) are combined in one unit (234).

12. The device according to one of claims 1 to 11, **characterized in that** the device detects an effect of substances other than sodium on the measured conductivity when measuring the temperature-compensating conductivity and corrects the measured conductivity according to an existence of the other substances.

13. A method for attaching the device according to claims 1 to 12 to the dialysis machine comprising:

- attaching the first measuring device (14) to the discharge line (13) of the dialysis machine,
- optionally attaching an optional fourth measuring device (15) to the discharge line (13) of the dialysis machine,
- if applicable, attaching the second measuring device (11) to the supply line (10) of the dialysis machine,
- if applicable, attaching an optional third measuring device (12) to the supply line (10) of the dialysis machine.

14. The method according to claim 13, **characterized in that** the sensor unit (2) is invasively attached to the conduit (102, 103) by:

- separating the line into two parts,
- connecting the sensor unit (2) to the first part, and
- connecting the sensor unit (2) to the second part.

**Revendications**

1. Dispositif de bilan électrolytique pour la mise à niveau d'une machine de traitement extracorporel du sang (100), en particulier d'une machine d'hémodialyse, présentant :

    - une unité de détection (2), avec :
    - au moins un premier dispositif de mesure (14) pour détecter une teneur en sodium d'un liquide de dialyse usé,
    - une section ou partie de liaison (7a, 8a) pour relier, de préférence de manière adaptative, l'unité de détection (2) au circuit de fluide d'une machine de traitement du sang (100), de sorte que le premier dispositif de mesure (14) est adapté pour détecter la teneur en sodium, et
    - une unité électronique et d'évaluation (3) fonctionnant indépendamment de la machine de traitement extracorporel du sang (100), qui analyse la teneur en sodium détectée par le premier dispositif de mesure (14) et détermine, en fonction de la teneur en sodium détectée, une recommandation pour une teneur en sodium d'un liquide de dialyse frais et
    - une interface d'entrée-sortie (4), de préférence combinée, qui est conçue pour délivrer à un utilisateur la recommandation déterminée par l'unité électronique et d'évaluation (3) pour la teneur en sodium du liquide de dialyse frais, et
    - une alimentation en énergie (9) réalisée indépendamment de la machine de traitement extracorporel du sang (100).

2. Dispositif selon la revendication 1, **caractérisé par**
    une unité de mémoire lisible par une machine, sur laquelle est stocké un programme comprenant les étapes suivantes consistant à :

    - initialiser la détection d'une grandeur corrélée à la concentration de sodium plasmatique dans le sang du patient par le premier dispositif de mesure (14) par l'utilisateur/opérateur via l'unité de commande (4) ;
    - détecter la teneur en sodium du liquide de dialyse usé par le premier dispositif de mesure (14) ;
    - ordonner à l'utilisateur/opérateur, par l'intermédiaire de l'unité de commande (4), de commuter la machine de traitement du sang (100) dans un mode de dérivation ;
    - informer le dispositif du mode de dérivation, par exemple par une détection sensorielle autonome ou par une entrée manuelle sur l'unité de commande (4) ;
    - donner à l'utilisateur/opérateur l'instruction, par l'intermédiaire de l'unité de commande (4), de commuter la machine de traitement du sang (100) dans le mode de fonctionnement normal ;
    - informer le dispositif du mode de fonctionnement normal, par exemple par une détection sensorielle autonome ou par une entrée manuelle sur l'unité de commande (4) ;
    - détecter la teneur en sodium du liquide de dialyse usé par le premier dispositif de mesure (14) ;
    - analyser la teneur en sodium détectée du liquide de dialyse usé par l'unité électronique et d'évaluation (3) et émettre la recommandation d'une teneur en sodium du liquide de dialyse frais en fonction de la teneur en sodium détectée dans le liquide de dialyse usé via l'unité de commande (4) ;
    - donner à l'utilisateur/opérateur l'instruction, par l'intermédiaire de l'unité de commande (4), de régler la teneur en sodium recommandée sur la machine de traitement du sang (100).

3. Dispositif selon la revendication 1 ou 2, **caractérisé par** un deuxième dispositif de mesure (11) qui est prévu dans une conduite d'alimentation (10) et qui détecte la teneur en sodium du liquide de dialyse frais.

4. Dispositif selon la revendication 3, **caractérisé en ce que** le premier dispositif de mesure (14) et le deuxième dispositif de mesure (11) peuvent effectuer des mesures à la fois de manière invasive et non invasive.

5. Dispositif selon la revendication 4, **caractérisé en ce que** le premier dispositif de mesure (14) et le deuxième dispositif de mesure (11) déterminent une conductivité du liquide de dialyse compensée en température.

6. Dispositif selon la revendication 3 ou 4, **caractérisé en ce que** le premier dispositif de mesure (14) et le deuxième dispositif de mesure (11) déterminent une concentration d'ions dans le liquide de dialyse.

7. Dispositif selon l'une quelconque des revendications 1 à 6, **caractérisé en ce que** le dispositif présente trois valves par lesquelles le mode de dérivation est réglé sur le dispositif, dans lequel, dans le mode de dérivation, au moins une valve d'entrée est fermée et au moins une valve est ouverte pour guider le liquide de dialyse autour d'un dialyseur.

8. Dispositif selon l'une quelconque des revendications 1 à 7, **caractérisé en ce que** la recommandation pour la teneur en sodium du liquide de dialyse est choisie par l'unité électronique et d'évaluation (3) de sorte qu'une concentration en sodium plasmatique dans le sang d'un patient reste sensiblement constante au cours d'une durée du traitement du sang.

9. Dispositif selon l'une quelconque des revendications 1 à 8, **caractérisé par** un autre dispositif de mesure (15) qui détermine une grandeur chimique ou physique, de préférence une propriété d'absorption telle qu'une absorbance, en particulier une teneur en urée, du liquide de dialyse usé dans la conduite d'évacuation (13).

10. Dispositif selon la revendication 9, **caractérisé en ce que** les dispositifs de mesure (11, 14, 15) sont montés individuellement directement sur les conduites respectives (10, 13).

11. Dispositif selon l'une quelconque des revendications 1 à 10, **caractérisé en ce que** l'unité de détection (2), l'unité électronique et d'évaluation (3) et l'unité de commande (4) sont réunies dans une unité (234).

12. Dispositif selon l'une quelconque des revendications 1 à 11, **caractérisé en ce que**, lors de la mesure de la conductivité compensée en température, le dispositif détecte un effet de substances autres que le sodium sur la conductivité mesurée et corrige la conductivité mesurée en fonction d'une existence des autres substances.

13. Procédé d'installation du dispositif selon les revendications 1 à 12 sur la machine de dialyse, présentant les étapes consistant à :

    - installer le premier dispositif de mesure (14) sur la conduite d'évacuation (13) de la machine de dialyse,
    - le cas échéant, installer un quatrième dispositif de mesure (15) facultatif sur la conduite d'évacuation (13) de la machine de dialyse,
    - le cas échéant, installer le deuxième dispositif de mesure (11) sur la conduite d'alimentation (10) de la machine de dialyse,
    - le cas échéant, installer un troisième dispositif de mesure (12) facultatif sur la conduite d'alimentation (10) de la machine de dialyse.

14. Procédé selon la revendication 13, **caractérisé en ce que** l'unité de capteur (2) est montée de manière invasive sur la conduite (102, 103) de la façon suivante :

    - la conduite est séparée en deux parties,
    - l'unité de capteur (2) est reliée à la première partie, et
    - l'unité de capteur (2) est reliée à la seconde partie.

Fig. 1

Fig. 2

Fig. 3

Fig. 4

Fig. 5

EP 4 153 263 B1

Fig. 6

Fig. 7

Fig. 8

Fig. 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0097366 A2
- WO 2010112223 A1
- DE 102017116097 A1
- DE 102014116415 A1

**In der Beschreibung aufgeführte Nicht-Patentliteratur**

- **FLYTHE, J. E. ; KIMMEL, S. E. ; BRUNELLI, S. M.** Rapid fluid removal during dialysis is associated with cardiovascular morbidity and mortality. *Kidney International,* 2010, vol. 79, 250-257
- **SARAN, R. ; BRAGG-GRESHAM, J ; LEVIN, N. ; TWARDOWSKI, Z. ; WIZEMANN, V. ; SAITO, A. ; KIMATA, N. ; GILLESPIE, B.** Longer treatment time and slower ultrafiltration in hemodialysis: Associations with reduced mortality in the DOPPS. *Kidney International,* 2006, vol. 69, 1222-1228
- **PAULA, F. M. ; PEIXOTO, A. J. ; PINTO, L. V. ; DORIGO, D. ; PATRICIO, P. J. M. ; SANTOS, S. F. F.** Clinical consequences of an individualized dialysate sodium Prescription in hemodialysis patients. *Kidney International,* 2004, vol. 66, 1232-1238
- **BASILE, C. ; LOMONTE, C.** It is Time to Individualize the Dialysate Sodium Prescription. *Seminars in Dialysis,* 2016, vol. 29, 24-27
- **MENDOZA, J. M. ; SUN, S. ; CHERTOW, G. M. ; MORAN, J. ; DASS, S. ; SCHILLER, B.** Dialysate Sodium and Sodium gradient in maintenance hemodialysis: a neglected sodium restriction approach?. *Nephrol Dial Transplant,* 2011, vol. 26, 1281-1287
- Zero Diffusive Sodium Balance in Hemodialysis Provided by an Algorithm-Based Electrolyte Balancing Controller: A Proof of Principle Clinical Study. **KUHLMANN, U. ; MAIERHOFER, A. ; CANAUD, B. ; HOYER, J. ; GROSS, M.** Artificial Organs. Wiley, 2018